Europäisches Patentamt

European Patent Office

Office européen des brevets

(19)

(11) **EP 0 662 009 B1**

(12) **FASCICULE DE BREVET EUROPEEN**

(45) Date de publication et mention
de la délivrance du brevet:
**03.06.1998 Bulletin 1998/23**

(21) Numéro de dépôt: 93920898.9

(22) Date de dépôt: 17.09.1993

(51) Int. Cl.$^6$: **A61M 16/00**

(86) Numéro de dépôt international:
**PCT/FR93/00902**

(87) Numéro de publication internationale:
**WO 94/06499 (31.03.1994 Gazette 1994/08)**

(54) **DISPOSITIF D'AIDE A LA RESPIRATION**

ATEMHILFSGERÄT

BREATHING AID DEVICE

(84) Etats contractants désignés:
**AT BE CH DE DK ES GB IE IT LI NL SE**

(30) Priorité: **18.09.1992 FR 9211131**

(43) Date de publication de la demande:
**12.07.1995 Bulletin 1995/28**

(73) Titulaire:
**NELLCOR PURITAN BENNETT FRANCE
DEVELOPPEMENT
91975 Courtaboeuf-Les Ulis Cédex (FR)**

(72) Inventeur: **BOURDON, Guy
F-91370 Verrières-le-Buisson (FR)**

(74) Mandataire: **Pontet, Bernard et al
Pontet & Allano S.E.L.A.R.L.
25, rue Jean-Rostand
Parc Club Orsay Université
91893 Orsay Cédex (FR)**

(56) Documents cités:
| | |
|---|---|
| **EP-A- 0 042 321** | **EP-A- 0 283 141** |
| **EP-A- 0 521 314** | **FR-A- 1 492 136** |
| **FR-A- 2 159 735** | **FR-A- 2 291 739** |
| **FR-A- 2 682 042** | **US-A- 4 050 458** |
| **US-A- 4 466 433** | **US-A- 4 838 257** |
| **US-A- 5 000 173** | |

Printed by Xerox (UK) Business Services
2.16.3/3.4

## Description

La présente invention concerne un dispositif d'aide respiratoire, encore appelé "dispositif de ventilation" ou "ventilateur".

La ventilation par aide inspiratoire est un mode de ventilation partielle bien connu en réanimation.

Dans la suite, on appelle "ventilation" les échanges respiratoires provoqués et/ou assistés par un appareil. On appellera "circuit patient" des éléments de canalisation, tels qu'un masque, un conduit, etc., qui relient les voies aériennes du patient avec les moyens de production, du flux gazeux dans l'appareil d'aide respiratoire. Les appareils visés par l'invention sont destinés à aider par une légère surpression à l'inspiration, les patients qui ont certes des difficultés respiratoires, mais conservent une activité et une cadence_respiratoires qu'il s'agit de respecter.

L'aide inspiratoire consiste à appliquer pendant la phase inspiratoire, initiée en principe par le patient, une pression positive constante dans le circuit patient d'un appareil respiratoire.

Dans les appareils de ventilation par aide inspiratoire, l'expiration, également initiée par le patient, est passive, et s'effectue à la pression atmosphérique ou sous une pression expiratoire positive, encore appelée PEP.

Les ventilateurs utilisés en réanimation sont des machines complexes, comportant plusieurs modes de ventilation, fonctionnant à partir de gaz comprimés et adaptés à des patients intubés ou trachéotomisés, c'est-à-dire munis d'une canule de respiration introduite dans la trachée artère par le nez ou respectivement par une incision dans le cou.

On connaît d'après le EP-B-317417, un dispositif d'aide respiratoire dans lequel le circuit patient comprend une valve d'expiration à commande pneumatique. Pendant la phase d'inspiration, l'entrée de commande de cette valve est soumise à la pression d'une source de débit pressurisé, ce qui ferme la valve d'expiration et par conséquent relie de manière étanche le circuit patient avec la source de débit pressurisé. Lorsque l'on détecte une diminution significative du débit inspiré, une électronique de commande interrompt le fonctionnement de la source de débit pressurisé, dont la structure est telle que son orifice de sortie se trouve alors ramené à la pression atmosphérique. Cette pression est donc appliquée à la valve d'expiration, ce qui permet à celle-ci de s'ouvrir.

Ce dispositif ne peut fonctionner que sous pression expiratoire égale à la pression atmosphérique.

On connaît par ailleurs d'après le EP-A-0425092 un dispositif d'aide à la respiration comportant, à la place de la valve d'expiration, un orifice de fuite permanente calibré, tandis que l'on règle à deux niveaux différents la pression d'une source de débit, selon que l'on se trouve en phase d'inspiration ou d'expiration.

Ce dispositif ne peut fonctionner que sous pression d'expiration positive de façon que le sens de l'écoulement entre la source de débit et le patient soit toujours orienté de la source vers le patient, même pendant les phases d'expiration, pour éviter que du gaz expiratoire ne remonte vers la source de débit pendant les phases expiratoires pour être réinspiré lors de la phase inspiratoire suivante.

Par ailleurs, on connaît d'après le FR-A-2 291 739, le EP-A-0 042 321 des appareils volumétriques imposant au patient les volumes et les cadences respiratoires à des patients qui n'ont plus de réflexe respiratoire.

Le FR-A-1 492 136 impose lui aussi la cadence de respiration.

Le US-A-4 838 257 commande, de manière analogue, les formes d'onde imposées au patient.

Les trois premiers de ces documents décrivent une valve de distribution disposée dans le circuit d'inspiration et commandée pour être fermée pendant l'expiration. Mais il s'agit d'une valve pilotée d'après des paramètres propres à l'appareil de respiration lui-même pour obliger un patient en état comateux à respirer selon un cycle déterminé et avec des volumes déterminés.

Le but de l'invention est ainsi de proposer un dispositif d'aide respiratoire dont la structure de base soit compatible aussi bien avec l'expiration sous pression atmosphérique qu'avec l'expiration sous pression expiratoire positive, tout en étant relativement économique et compact, notamment convenir pour le fonctionnement au domicile du patient.

Suivant l'invention, le dispositif d'aide respiratoire comprenant un circuit patient ayant une branche inspiratoire reliée à une source de débit inspiratoire pressurisé et une branche expiratoire dans laquelle est installée une valve d'expiration qui est commandée pour être fermée pendant l'inspiration, le dispositif comprenant en outre des moyens de pilotage reliés à au moins un capteur détectant l'activité respiratoire du patient et des moyens de distribution qui en phase inspiratoire établissent la communication entre la source de débit inspiratoire et la branche inspiratoire du circuit patient, et en phase expiratoire interrompent au moins partiellement cette communication, caractérisé en ce que les moyens de distribution sont commandés par les moyens de pilotage, lesquels commandent l'interruption de ladite communication lorsque le capteur détecte que le patient prépare une phase expiratoire, et en ce que les moyens de pilotage commandent en outre, au moins indirectement, la valve d'expiration pour qu'elle détermine une pression expiratoire prédéfinie sensiblement indépendante de l'état de fonctionnement de la source de débit inspiratoire.

Selon l'invention, on ne commande plus la source de débit inspiratoire de l'appareil d'aide pour la faire fonctionner de manière particulière lors de l'expiration du patient, mais on commande une interruption de la communication entre la source de débit inspiratoire et le circuit patient, et on règle la pression d'expiration par des moyens indépendants de l'état de fonctionnement de la source de débit inspiratoire. Cette interruption de communication a l'originalité, même par rapport aux appareils de respiration forcée, d'être commandée principalement par l'activité respiratoire du malade, les moyens de commande de l'appareil ne servant que de relais.

L'interruption de communication entre la source de débit inspiratoire et la branche inspiratoire pendant l'expiration empêche la branche inspiratoire d'accumuler les gaz expirés. Ceci supprime le risque que de grandes quantités de gaz expirés soient ensuite réinhalées. On n'a donc plus à tenir compte du problème de réinhalation pour définir la pression à laquelle le patient est soumis pendant l'expiration.

De plus, la séparation entre la commande de la valve d'expiration et l'état de fonctionnement de la source de débit inspiratoire pendant les phases expiratoires permet de définir la pression expiratoire sans avoir non plus à tenir compte des impératifs de fonctionnement de la source de débit inspiratoire. Par conséquent, l'invention permet de choisir librement la pression expiratoire.

La notion d'indépendance entre la pression d'expiration définie par la valve d'expiration et l'état de fonctionnement de la source de débit inspiratoire doit s'apprécier de manière relative, et notamment au vu de l'état de la technique. D'après le EP-B-317417, on est obligé de modifier le fonctionnement de la source de débit inspiratoire pour que la valve d'expiration modifie son fonctionnement lors des transitions entre les phases respiratoires.

Au contraire, selon l'invention, on se libère de cette relation contraignante. Mais ceci n'exclut pas, par exemple, que la valve d'expiration soit du type à commande pneumatique, et soit commandée par une source de pression réalisée à partir d'une dérivation calibrée reliée à la sortie de la source de débit inspiratoire, comme on le verra dans un exemple décrit plus loin. Dans ce cas non limitatif, le calibrage crée l'indépendance puisqu'on peut choisir le calibrage pour que la valve d'expiration assure la pression expiratoire que l'on souhaite.

De préférence, la source de débit inspiratoire est une source dont le débit tend à s'annuler lorsque la pression à sa sortie prend une valeur maximale qui est de l'ordre de grandeur d'une pression d'aide à l'inspiration.

Ainsi, lorsque pendant une phase expiratoire les moyens de distribution interrompent au moins partiellement la communication entre la source de débit inspiratoire et la branche inspiratoire du circuit patient, la source de débit inspiratoire ne nécessite pas de pilotage particulier: elle continue a fonctionner à débit nul, avec simplement une légère élévation de sa pression.

Il est avantageux que, comme évoqué plus haut, la valve d'expiration soit du type à commande pneumatique. Pour qu'elle détermine la pression expiratoire, les moyens de pilotage relient son entrée de commande à une source de basse pression. Celle-ci peut comprendre une liaison entre la sortie d'une deuxième source de débit et un ajutage d'échappement. Le réglage du débit de cette deuxième source de débit permet de régler la pression appliquée à l'entrée de commande de la valve d'expiration et permet par conséquent de régler la pression sous laquelle le patient devra expirer pour pouvoir provoquer l'ouverture de la valve d'expiration. Si le débit de la deuxième source de débit est réglé à la valeur nulle, l'entrée de commande est en communication avec l'atmosphère via l'ajutage d'échappement et par conséquent la pression expiratoire est égale à la pression atmosphérique.

D'autres particularités et avantages de l'invention ressortiront encore de la description ci-après, relative à des exemples non limitatifs.

Aux dessins annexés:

- la figure 1 est un schéma d'un premier mode de réalisation du dispositif selon l'invention;
- la figure 2 est un organigramme de commande du dispositif de la figure 1;
- la figure 3 est un schéma d'un mode de réalisation plus particulier du dispositif selon la figure 1;
- la figure 4 est un schéma simplifié relatif à une variante ; et
- la figure 5 est un schéma analogue à la figure 3, mais relatif à une variante.

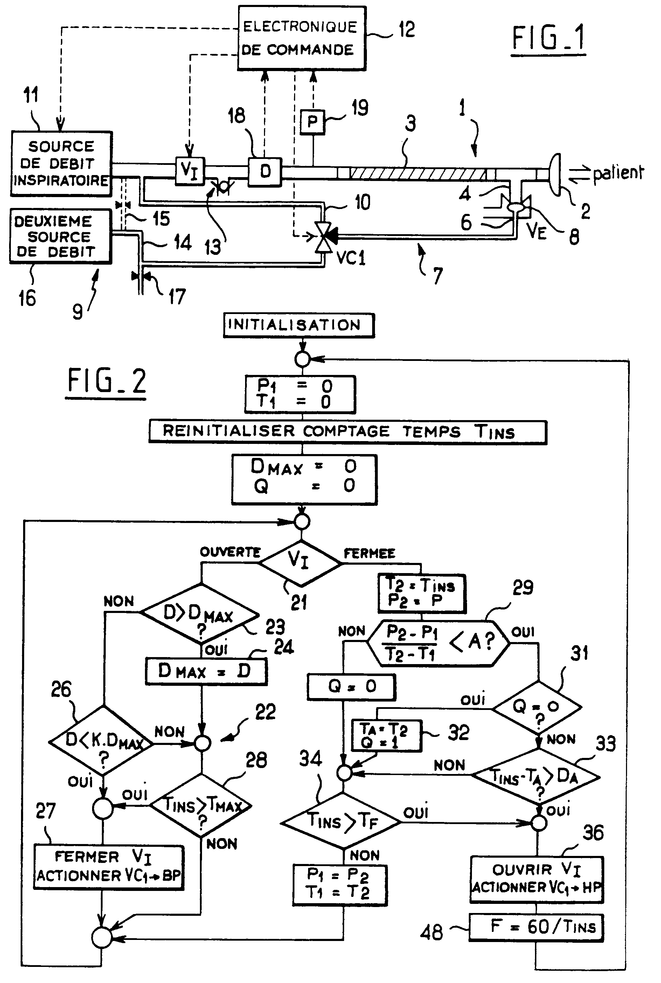

Dans l'exemple représenté à la figure 1, le dispositif d'aide respiratoire comprend un circuit patient 1 comprenant à son tour un masque facial, nasal ou buccal 2, raccordé à une branche inspiratoire 3 et à une branche expiratoire 4. La branche expiratoire 4 comporte une valve d'expiration $V_E$ du type à commande pneumatique comportant une entrée de commande 6 reliée à un circuit de commande 7.

La valve d'expiration $V_E$ comporte un obturateur 8 qui ferme la valve si la pression relative à l'entrée de commande 6 est au moins égale à une fraction prédéterminée de la pression relative présente à l'entrée de la valve, située ici du côté du masque 2. L'obturateur 8 est par exemple constitué par une enceinte gonflable soumise à la pression de l'entrée de commande 6, ou encore par une membrane dont la face opposée à l'entrée de la valve est soumise à la pression régnant à l'entrée de commande 6.

Pendant les phases expiratoires de la respiration du patient, la branche expiratoire 4 communique avec l'atmosphère à condition que le patient produise une pression expiratoire suffisante pour ouvrir la valve d'expiration $V_E$, dont l'entrée de commande 6 est alors reliée d'une manière que l'on explicitera plus loin avec une source de basse pression 9. D'après les indications qui précèdent, si la pression relative produite par la source de basse pression 9 est nulle, c'est-à-dire égale à la pression atmosphérique, la valve d'expiration $V_E$ s'ouvre sans que le patient ait à fournir une pression expiratoire significativement supérieure à la pression atmosphérique. Au contraire, si la pression relative de la source 9 est supérieure à zéro, le patient devra lui-même fournir une certaine pression relative pour expirer.

La branche inspiratoire 3 du circuit patient 1 est reliée à la sortie d'une source de débit inspiratoire pressurisé 11 qui peut être constituée par un groupe moto-turbine, un ensemble éjecteur ou venturi alimenté par un compresseur ou une bouteille de gaz comprimé, etc.

Des moyens de distribution, comprenant une valve d'inspiration $V_I$ interposée entre la sortie de la source de débit inspiratoire et la branche inspiratoire 3 du circuit patient 1, commandent les échanges gazeux tels qu'initiés par le patient. Suivant l'invention, la valve d'inspiration $V_I$ est pilotée par l' électronique de commande 12 pour mettre la sortie de la source de débit inspiratoire en communication avec la branche inspiratoire 3 pendant les phases inspiratoires de la respiration du patient, et pour interrompre cette communication pendant les phases expiratoires.

La source de débit inspiratoire 11 est d'un type capable d'annuler son débit avec seulement une légère augmentation de pression lorsque sa sortie est obturée. Ainsi, il n'est pas nécessaire de modifier les commandes appliquées à la source de débit inspiratoire 11 selon que la vanne d'inspiration $V_I$ est ouverte ou fermée. Si la source de débit inspiratoire est un groupe moto-turbine, ses caractéristiques peuvent être les suivantes :

- pression de refoulement à vitesse maximum : 45 cm $H_2O$ (4,5kPa),
- débit à vitesse maximum et pression de sortie nulle : 280 à 300 l/mn.

Dans un but de sécurité, il est prévu en aval de la valve d'inspiration $V_I$ un clapet anti-retour 13 qui fait communiquer la branche inspiratoire 3 avec l'atmosphère lorsque la pression dans la branche inspiratoire 3 devient inférieure à la pression atmosphérique. Ainsi, le patient ne serait pas privé d'air frais au cas où une panne maintiendrait la valve d'inspiration $V_I$ en position de fermeture.

L'électronique de commande 12 pilote une vanne de commande VC1 en synchronisme avec la valve d'inspiration $V_I$. La vanne de commande VC1, du type à trois voies, est installée dans le circuit de commande 7 de la valve d'expiration $V_E$. Lorsque la valve d'inspiration $V_I$ est fermée, la vanne de commande VC1 relie l'entrée de commande 6 de la valve d'expiration $V_E$ avec la source de basse pression 9. Lorsque la valve $V_I$ est ouverte, la vanne de commande VC1 relie l'entrée de commande 6 avec la sortie de la source de débit inspiratoire pressurisé 11.

La source de basse pression 9 est constituée par une liaison 14 entre une deuxième source de débit 16 et un ajutage 17 d'échappement vers l'atmosphère. La deuxième source de débit est réglable dans une plage commençant à la valeur nulle. Dans ce cas, la pression dans la liaison 14 est rendue égale à la pression atmosphérique à travers l'ajutage 17 et par conséquent, en phase d'expiration, la pression imposée au patient est également la pression atmosphérique. Au contraire, si le débit de la deuxième source 16 est non nul, une certaine pression, qui est fonction de ce débit, s'établit dans la liaison 14 et impose par conséquent une pression expiratoire positive au patient. Les pressions produites par la source de basse pression 9 sont inférieures à la pression produite par la source de débit inspiratoire 11.

Lorsque la valve d'inspiration $V_I$ est ouverte, la vanne de commande VC1 relie l'entrée de commande 6 de la valve d'expiration $V_E$ par un conduit 10 avec la pression de la source de débit inspiratoire en amont de la valve d'inspiration $V_I$. Compte tenu de ce qui a été dit plus haut sur le fonctionnement de la valve d'expiration $V_E$, une telle pression provoque la fermeture de la valve d'expiration $V_E$ et par conséquent le masque 2 est relié de manière étanche avec la sortie de la source de débit inspiratoire.

Pour assurer la transition du mode expiratoire au mode inspiratoire et inversement, l'électronique de commande 12 reçoit des signaux de mesure produits par un débitmètre 18 et un manomètre 19 fournissant des données de débit D et de pression P de l'écoulement à travers la branche inspiratoire 3.

On va maintenant décrire plus en détail, en référence à la figure 2, certaines particularités de fonctionnement et d'automatisme de la figure 1.

Lorsqu'un test 21 détecte que la vanne d'inspiration $V_I$ est ouverte, on surveille, dans une partie 22 de l'organigramme, l'évolution du débit inspiratoire. On sait que le débit inspiratoire atteint rapidement un maximum $D_{MAX}$ au début de chaque phase inspiratoire. Par le test 23 et l'étape 24, on réactualise la valeur du débit maximum $D_{MAX}$ tant que le débit augmente.

Au contraire, lorsque le débit inspiratoire commence à diminuer, on est conduit à un test 26 qui détermine si le débit instantané D est ou non devenu inférieur à une certaine fraction (coefficient K égal par exemple à 0,6) du débit maximum $D_{MAX}$. Dans l'affirmative, l'électronique de commande 12 décide que la phase inspiratoire est terminée et elle commande la fermeture de la valve d'inspiration $V_I$ et l'actionnement de la vanne de commande VC1 pour la relier à la source de basse pression 9. Si la réponse au test 26 est négative, on vérifie encore par un test 28 si la durée de l'inspiration n'excède pas une durée maximale $T_{MAX}$, fixée arbitrairement par exemple à 3 secondes. Si oui, on prend également une décision de fin de phase d'inspiration par l'étape 27.

On est ensuite conduit de nouveau à l'entrée du test sur l'état de la valve d'inspiration $V_I$.

Lorsque cette valve d'inspiration est fermée, on surveille par un test 29 l'apparition, dans le diagramme de pression en fonction du temps, d'une dérivée ou pente $(P_2-P_1)/(T_2-T_1)$ qui soit inférieure à une valeur négative prédéterminée A. En effet, une baisse de la pression à la fin d'une phase d'expiration est significative d'un appel d'air de la part du patient. Si une telle pente négative est détectée, un test 31 sur un paramètre logique Q permet de savoir s'il s'agit de la première itération où la pente négative est détectée au cours de ce cycle expiratoire. Si oui, par une étape 32, on met à 1 le paramètre logique Q et on relève sous le registre $T_A$ l'instant $T_2$ de début de la pente inférieure à A. Dans les itérations suivantes (sortie négative du test 31), on va rechercher par un test 33 la durée de cette pente négative. Lorsque cette durée devient supérieure à une durée d'appel préétablie $D_A$, l'électronique décide la fin de la phase d'expiration, c'est-à-dire l'ouverture de la valve d'inspiration $V_I$ et l'actionnement de la vanne de commande $VC_1$ pour relier l'entrée de commande 6 de la valve d'expiration $V_E$ avec la sortie de la source de débit inspiratoire 11.

Tant que la sortie du test 29 ou la sortie du test 33 est négative, on surveille par un test 34 l'évolution du temps $T_{INS}$ écoulé depuis le début de la phase d'inspiration précédente. Si cette durée devient supérieure à une période respiratoire maximale prédéterminée $T_F$, l'électronique de commande passe à l'étape 36 de fin de l'expiration.

Selon une variante représentée en pointillés à la figure 1, la deuxième source de débit 16 peut être matérialisée par un conduit calibré 15 reliant la liaison 14 avec la sortie de la source de débit inspiratoire. Pour faire varier le débit à travers le conduit 15, le calibrage peut être assuré par un robinet réglable jusqu'à une position de fermeture totale. Même dans ce mode de réalisation, la source de basse pression est dite "indépendante" ou "séparée" de la source de débit inspiratoire, en ce sens que la pression de la source de basse pression, et par conséquent le signal de basse pression appliqué à l'entrée de commande 6 de la valve d'expiration $V_E$, prennent la valeur souhaitée pour l'expiration sans que le fonctionnement de la source de débit inspiratoire n'ait à être modifié dans ce but.

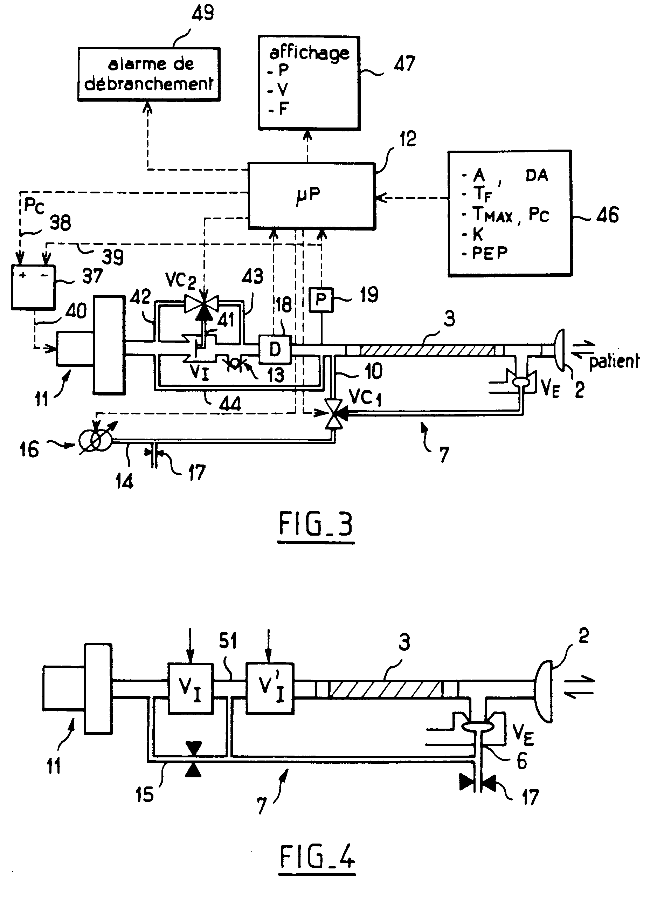

Dans l'exemple représenté à la figure 3, la source de débit inspiratoire 11 est constituée par un groupe moto-turbine alimenté par un variateur de vitesse 37 recevant sur son entrée positive 38 un signal de consigne de pression PC et sur son entrée négative 39 un signal issu du détecteur de pression 19. En fonction de l'écart entre ses deux entrées 38 et 39, le variateur de vitesse 37 délivre au groupe moto-turbine 11 sur sa sortie 40 un signal de puissance approprié pour tendre à ramener en permanence la pression P à la valeur de consigne Pc pendant les phases inspiratoires. De manière non représentée, pendant les phases expiratoires, le variateur 37 est court-circuité et le micro-processeur envoie au groupe moto-turbine un signal maintenant sensiblement le groupe moto-turbine à la vitesse qu'il avait pendant l'inspiration précédente.

La valve d'inspiration $V_I$ est constituée par une valve à commande pneumatique d'une structure pouvant être semblable à la valve d'expiration $V_E$. En particulier, la valve $V_I$ possède une entrée de commande 41 qui est soumise en service à une pression qui détermine l'état ouvert ou fermé de la valve.

La valve d'inspiration $V_I$ est associée à une vanne de commande $VC_2$ qui est du type à trois voies pour relier sélectivement l'entrée de commande 41 avec un conduit 42 relié à la sortie du groupe moto-turbine 11 pour réaliser la fermeture de la valve d'inspiration, ou avec un conduit 43 relié avec le trajet de gaz inspirable en aval de la valve $V_I$ pour réaliser l'état ouvert de la valve d'inspiration $V_I$. On comprend que la pression en aval de la valve d'inspiration $V_I$ s'établit nécessairement à une valeur d'équilibre permettant l'ouverture de la valve car si la valve se fermait, la pression relative en aval disparaîtrait et par conséquent la valve se réouvrirait aussitôt de manière importante.

Par ailleurs, la valve d'inspiration $V_I$, le clapet de sécurité 13, le raccordement du conduit 43 avec le trajet principal d'inspiration et le débitmètre 18 sont court-circuités par un trajet de compensation de fuite 44 raccordant la sortie du groupe moto-turbine 11 avec la branche inspiratoire 3. Le rôle de ce conduit 44 est de compenser les fuites qui pourraient exister par exemple entre le masque 2 et le visage du patient pendant la phase expiratoire. En effet, une telle fuite peut empêcher le maintien de la pression expiratoire positive éventuellement imposée par la deuxième source débit 16.

La deuxième source de débit 16 est constituée par un compresseur à débit variable commandé selon une consigne appliquée par l'électronique de commande 12, elle-même réalisée sous la forme d'un microprocesseur.

Par ailleurs, dans l'exemple de la figure 3, le conduit 10 est raccordé à l'entrée de la branche inspiratoire 3, c'est-à-dire, notamment, en aval de la valve d'inspiration $V_I$ et du débitmètre 8.

Le microprocesseur 12 reçoit en entrée, par exemple au moyen d'un clavier non représenté, divers réglages 46 portant notamment sur les paramètres A, DA, $T_F$, $T_{MAX}$, K, apparaissant dans l'organigramme de la figure 2, ainsi que le paramètre PC représentant la consigne de pression appliquée à l'entrée 38 du variateur de vitesse 37, et la pression expiratoire positive PEP, servant à commander le compresseur 16.

Le microprocesseur 12 commande un dispositif d'affichage 47 permettant d'afficher la pression P mesurée par le capteur 19, le volume V de chaque inspiration, calculé par le microprocesseur d'après les signaux fournis par le manomètre 19 et le débitmètre 18, et la fréquence respiratoire F calculée à l'étape 48, en cycles par minute, à la figure 2.

Si le débit relevé par le débitmètre conserve une valeur maximale pendant une durée prédéterminée, cela est détecté par le microprocesseur 12 qui actionne une alarme de débranchement 49, sonore et/ou visuelle, pour signaler qu'un incident du genre détachement du masque 2 s'est produit.

Dans l'exemple représenté à la figure 4, qui ne sera décrit que pour ses différences par rapport à celui de la figure 1, la valve d'inspiration $V_I$ est suivie par une deuxième valve d'inspiration $V'_I$ qui peut être ouverte et fermée en même temps que la valve $V_I$, ou qui peut encore être constituée par un clapet anti-retour empêchant le gaz d'aller de la branche inspiratoire 3 vers le groupe moto-turbine 11. L'entrée de commande 6 de la valve d'expiration $V_E$ est reliée directement à un orifice d'échappement calibré 17, d'autre part à la partie 51 du trajet inspiratoire qui est situé entre les deux valves d'inspiration $V_I$ et $V'_I$. L'entrée de commande 6 est également reliée directement à la source de basse pression constituée ici par une liaison calibrée 15 avec la sortie du groupe moto-turbine 11.

Le fonctionnement est le suivant:

Quand les deux valves d'inspiration $V_I$ et $V'_I$ sont ouvertes, l'entrée de commande 6 de la valve d'expiration 2 est soumise à la pression inspiratoire fournie par le groupe moto-turbine 11 grâce à la liaison avec la partie 51 du trajet inspiratoire. La valve $V_E$ est donc fermée et l'orifice calibré 17 maintient la différence de pression entre la pression inspiratoire et la pression atmosphérique.

Pour l'expiration, les deux valves $V_I$ et $V'_I$ sont fermées, et l'entrée de commande 6 est soumise à la pression définie par la liaison 15, ou encore, si la liaison 15 est fermée par un réglage, à la pression atmosphérique à travers l'orifice calibré 17. Le patient expire donc sous la pression déterminée de cette manière par la valve d'expiration $V_E$.

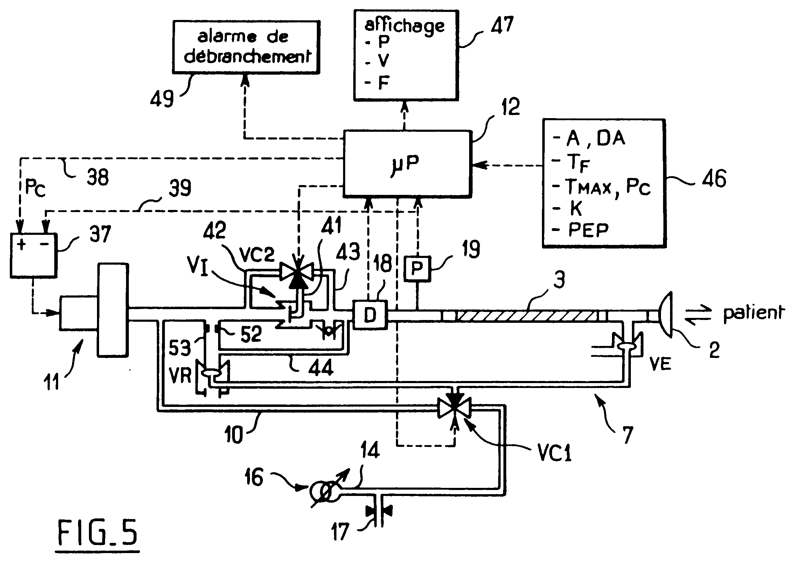

Le dispositif de la figure 5 ne sera décrit que pour ses différences par rapport à celui de la figure 3. Il comprend une valve supplémentaire $V_R$, reliant la sortie du groupe moto-turbine à l'atmosphère et commandée par le circuit de commande 7 de la valve expiratoire $V_E$ et donc par la même électrovanne VC1 que la valve expiratoire $V_E$. Une restriction 52 est placée dans une dérivation 53 raccordant la sortie du groupe moto-turbine 11 à la vanne supplémentaire $V_R$.

Le circuit de compensation de fuite 44 est connecté, d'une part entre la valve VR et la restriction 52, et d'autre part, en aval de la valve inspiratoire $V_I$.

Le fonctionnement est le suivant :

- pendant l'inspiration la valve VR est fermée car elle est commandée comme VE.
- pendant l'expiration, la valve VR maintient en son amont une pression égale à la pression d'expiration PEP car elle est commandée comme la valve VE par le mini-compresseur 16.

Il y a un débit qui part à l'atmosphère par la valve VR, car la pression à la sortie du groupe mototurbine 11 est supérieure à la pression d'expiration PEP. Ce débit sert à refroidir le groupe moto-turbine. Il y a en outre un débit qui part dans le circuit patient lorsque la pression dans celui-ci est inférieure à la pression d'expiration PEP, c'est à dire dans le cas d'une fuite.

Contrairement au dispositif de la figure 3, le débit de compensation de fuite n'est plus permanent, mais n'intervient que lorsqu'il y a une fuite.

La restriction 52 permet de maintenir à la sortie du groupe moto-turbine une pression importante (proche de la pression d'aide inspiratoire) nécessaire pour la fermeture de la valve inspiratoire VI, et de limiter le débit de refroidissement afin que la valve supplémentaire VR puisse réguler la pression en son amont.

Grâce à l'invention, on dispose d'un appareil simple, léger, ne nécessitant pas de bouteilles de gaz et permettant l'emploi à domicile dans une gamme de cas pathologiques très étendue.

Bien entendu, l'invention n'est pas limitée aux exemples décrits et représentés.

Par exemple, à la figure 3, au lieu de relier le conduit 43 de commande de l'ouverture de la valve d'inspiration $V_I$ avec le trajet de gaz inspiratoire, on pourrait le relier à la pression faible définie par la source de basse pression 14.

Dans les exemples, les termes "valve" et "vanne" sont appropriés pour définir les éléments $V_E$ et $V_I$, et respectivement $V_{C1}$ et $V_{C2}$. Mais ces termes ne doivent pas être interprétés limitativement, la "valve" $V_I$ pouvant notamment être réalisée sous la forme d'une vanne à commande mécanique ou électromécanique.

La valve d'expiration $V_E$ pourrait être d'un type autre qu'à commande pneumatique, par exemple à commande électrique.

## Revendications

1. Dispositif d'aide respiratoire du type à inspiration sous pression sensiblement constante, comprenant un circuit patient (1) ayant une branche inspiratoire (11) reliée à une source de débit inspiratoire pressurisé (3) et une branche expiratoire (4) dans laquelle est installée une valve d'expiration ($V_E$) qui est commandée pour être fermée pendant l'inspiration, le dispositif comprenant en outre des moyens de pilotage (12) reliés à au moins un capteur (18, 19) détectant l'activité respiratoire du patient et des moyens de distribution ($V_I$) qui en phase inspiratoire établissent la communication entre la source de débit inspiratoire (11) et la branche inspiratoire (3) du circuit patient, et en phase expiratoire interrompent au moins partiellement cette communication, caractérisé en ce que les moyens de distribution ($V_I$) sont commandés par les moyens de pilotage (12), lesquels commandent l'interruption de ladite communication lorsque le capteur détecte que le patient prépare une phase expiratoire, et en ce que les moyens de pilotage (12) commandent en outre, au moins indirectement, la valve d'expiration ($V_E$) pour qu'elle détermine une pression expiratoire prédéfinie sensiblement indépendante de l'état de fonctionnement de la source de débit inspiratoire (11).

2. Dispositif selon la revendication 1, caractérisé en ce que la source de débit inspiratoire est un groupe mototurbine (11).

3. Dispositif selon la revendication 1 ou 2, caractérisé en ce que pendant les phases expiratoires la source de débit inspiratoire (11) est maintenue en fonctionnement dans le sens tendant à fournir du débit inspiratoire.

4. Dispositif selon l'une des revendications 1 à 3, caractérisé en ce que la source de débit inspiratoire (11) est actionnée pour fournir une pression de sortie sensiblement égale pendant les phases inspiratoires et pendant les phases expiratoires.

5. Dispositif selon l'une des revendications 1 à 4, caractérisé en ce que la source de débit inspiratoire (11) est d'un type ayant une pression de refoulement qui reste du même ordre de grandeur sur une plage de valeurs de débit incluant les débits pendant les phases inspiratoires et les débits différents pendant les phases expiratoires.

6. Dispositif selon la revendication 5, caractérisé en ce que lesdits débits différents incluent un débit sensiblement nul.

7. Dispositif selon l'une des revendications 1 à 4, caractérisé et en ce que la source de débit inspiratoire (11) est une source dont le débit tend à s'annuler lorsque la pression à sa sortie prend une valeur maximale qui est de l'ordre de grandeur d'une pression d'aide à l'inspiration.

8. Dispositif selon l'une des revendications 1 à 7, caractérisé en ce que les moyens de distribution comprennent une valve d'inspiration ($V_I$) à commande pneumatique, dont une entrée de commande (41) est reliée sélectivement à la sortie de la source de débit inspiratoire (11) pendant l'expiration et à une pression plus faible (43) pendant l'inspiration.

9. Dispositif selon la revendication 8, caractérisé en ce que la pression plus faible est prélevée dans le trajet du gaz entre la valve d'inspiration ($V_I$) et le circuit patient (1).

10. Dispositif selon l'une des revendications 1 à 9, caractérisé en ce que les moyens de distribution sont conçus pour laisser subsister un débit de compensation de fuite (44) entre la source de débit inspiratoire (11) et le circuit patient (1) pendant l'expiration.

11. Dispositif selon l'une des revendications 1 à 9, caractérisé en ce que les moyens de distribution ($V_I$) sont contournés par une dérivation (53, 54) équipée d'une restriction (52) et, en aval de cette restriction, d'une vanne supplémentaire (VR) pilotée pour être fermée pendant l'inspiration et pour maintenir dans la dérivation en aval de la restriction (52), pendant l'expiration, une pression voisine de la pression expiratoire prédéfinie (PEP).

12. Dispositif selon l'une des revendications 1 à 11, caractérisé en ce que la valve d'expiration ($V_E$) est à commande pneumatique, et en ce que pour commander la valve d'expiration ($V_E$) les moyens de pilotage commandent l'activation d'un raccordement (7, VC1) entre une entrée de commande (6) de la valve d'expiration ($V_E$) et une source de basse pression (9, 15).

13. Dispositif selon la revendication 12, caractérisé en ce que le raccordement comprend des moyens de vannage (VC1) installés dans un circuit de commande (7) de la valve d'expiration ($V_E$) et commandés par les moyens de pilotage (12) pour relier l'entrée de commande (6) de la valve d'expiration avec la source de basse pression (14) pendant l'expiration du patient.

14. Dispositif selon la revendication 13, caractérisé en ce que les moyens de vannage comprennent une vanne à trois voies (VC1) reliant sélectivement l'entrée de commande (6) de la valve d'expiration ($V_E$) avec la source de débit pressurisé (11) et avec la source de basse pression (14).

15. Dispositif selon l'une des revendications 12 à 14, caractérisé en ce que la source de basse pression comprend une liaison (14) entre un ajutage d'échappement (17) et une sortie d'une deuxième source de débit (16).

16. Dispositif selon la revendication 15, caractérisé en ce que la deuxième source de débit comprend un compresseur (16).

17. Dispositif selon la revendication 15, caractérisé en ce que la deuxième source de débit comprend un dispositif (15) de liaison avec la source de débit inspiratoire (11).

18. Dispositif selon l'une des revendications 1 à 17, caractérisé en ce que la pression expiratoire prédéfinie est réglable et peut prendre une valeur égale à la pression atmosphérique.

19. Dispositif selon l'une des revendications 1 à 18, caractérisé en ce que les moyens de pilotage (12) commandent la fermeture de la valve d'expiration (V$_E$) lorsque la pression dans le circuit patient (1) diminue en fin de phase d'expiration du patient.

20. Dispositif selon la revendication 19, caractérisé en ce que les moyens de pilotage (12) sont sensibles à la dérivée de la pression dans le circuit patient (1) par rapport au temps.

21. Dispositif selon l'une des revendications 1 à 20, caractérisé en ce qu'il comprend des moyens (18, 19) de mesure de la pression (P) et du débit (D) dans la branche inspiratoire (3) du circuit patient (1), et des moyens de calcul du volume de gaz inspiré, ou d'une grandeur équivalente.

22. Dispositif selon l'une des revendications 1 à 21, caractérisé par des moyens (12) pour activer une alarme de débranchement (49) si le débit demeure élevé dans la branche inspiratoire (4) pendant une durée prédéterminée relativement grande.

**Claims**

1. Breathing aid device of the type with inspiration under substantially constant pressure, comprising a patient circuit (1) having an inspiratory branch (11) connected to a pressurized inspiratory flow source (3) and an expiratory branch (4) in which is installed an expiration valve (V$_E$) which is controlled so as to be closed during inspiration, the device also comprising driving means (12) connected to at least one sensor (18, 19) detecting the respiratory activity of the patient and distribution means (V$_I$) which in inspiratory phase establish the communication between the inspiratory flow source (11) and the inspiratory branch (3) of the patient circuit, and in expiratory phase interrupt this communication at least partially, characterized in that the distribution means (V$_I$) are controlled by the driving means (12), wich control the interruption of the said communication when the sensor detects that the patient is preparing an expiratory phase, and in that the driving means (12) also control, at least indirectly, the expiration valve (V$_E$) in order that it determines a predefined expiratory pressure substantially independent of the operating state of the inspiratory flow source (11).

2. Device according to claim 1, characterized in that the inspiratory flow source is a motor turbine group (11).

3. Device according to claim 1 or 2, characterized in that during the expiratory phases the inspiratory flow source (11) is kept in operation in the direction aiming to provide inspiratory flow.

4. Device according to one of claims 1 to 3, characterized in that the inspiratory flow source (11) is actuated in order to provide a substantially equal outlet pressure during the inspiratory phases and during the expiratory phases.

5. Device according to one of claims 1 to 4, characterized in that the inspiratory flow source (11) is of a type having a delivery pressure which remains of the same order of magnitude over a range of flow rate values including the flow rates during the inspiratory phases and the flow rates, which are different, during the expiratory phases.

6. Device according to claim 5, characterized in that the said different flow rates include a substantially zero flow rate.

7. Device according to one of claims 1 to 4, characterized in that the inspiratory flow source (11) is a source whose flow rate aims to cancel itself when the pressure at its outlet assumes a maximum value which is of the order of magnitude of an inspiration aid pressure.

8. Device according to one of claims 1 to 7, characterized in that the distribution means comprise a pneumatically controlled inspiration valve (V$_I$), a control inlet (41) of which is connected selectively to the outlet of the inspiratory flow source (11) during expiration and to a lower pressure (43) during inspiration.

9. Device according to claim 8, characterized in that the lower pressure is taken in the path of the gas between the inspiration valve (V$_I$) and the patient circuit (1).

10. Device according to one of claims 1 to 9, characterized in that the distribution means are designed to allow a leak compensation flow (44) to remain between the inspiratory flow source (11) and the patient circuit (1) during expiration.

11. Device according to one of claims 1 to 9, characterized in that the distribution means (V$_I$) are bypassed by a loop (53, 54) fitted with a restriction (52) and, downstream from this restriction, with a supplementary gate valve (VR) driven in order to be closed during inspiration and in order to maintain in the loop downstream from the restriction (52), during expiration, a pressure close to the predefined expiratory pressure (PEP).

12. Device according to one of claims 1 to 11, characterized in that the expiration valve (V$_E$) is pneumatically controlled, and in that, in order to control the expiration valve ($V_E$), the driving means control the actuation of a connection (7, VC1) between a control inlet (6) of the expiration valve ($V_E$) and a low pressure source (9, 15).

13. Device according to claim 12, characterized in that the connection comprises gating means (VC1) installed in a control circuit (7) of the expiration valve ($V_E$) and controlled by the driving means (12) in order to connect the control inlet (6) of the expiration valve to the low pressure source (14) when the patient is breathing out.

14. Device according to claim 13, characterized in that the gating means comprise a three-way gate valve (VC1) selectively connecting the control inlet (6) of the expiration valve ($V_E$) to the pressurized flow source (11) and to the low pressure source (14).

15. Device according to one of claims 12 to 14, characterized in that the low pressure source comprises a link (14) between an exhaust nozzle (17) and an outlet of a second flow source (16).

16. Device according to claim 15, characterized in that the second flow source comprises a compressor (16).

17. Device according to claim 15, characterized in that the second flow source comprises a device (15) linking with the inspiratory flow source (11).

18. Device according to one of claims 1 to 17, characterized in that the predefined expiratory pressure is adjustable and can assume a value equal to the atmospheric pressure.

19. Device according to one of claims 1 to 18, characterized in that the driving means (12) control the closure of the expiration valve ($V_E$) when the pressure in the patient circuit (1) decreases at the end of the patient's expiration phase.

20. Device according to claim 19, characterized in that the driving means (12) are reponsive to the variation in pressure in the patient circuit in relation to time.

21. Device according to one of claims 1 to 20, characterized in that it comprises means (18, 19) for measuring the pressure (P) and the flow rate (D) in the inspiratory branch (3) of the patient circuit, and means for calculating the volume of the inspired gas, or an equivalent variable.

22. Device according to one of claims 1 to 21, characterized by means for actuating a disconnection alarm if the flow rate remains high in the inspiratory branch (4) for a relatively great predetermined period.

## Patentansprüche

1. Beatmungsvorrichtung von der Art, bei der die Inspiration unter einem im wesentlichen gleichbleibenden Druck erfolgt, umfassend einen Patientenkreislauf (1) mit einem Inspirationszweig (11), der mit einer Quelle für einen Inspirations-Druckförderstrom (3) verbunden ist, und einem Exspirationszweig (4), in dem ein Exspirationsventil ($V_E$) angeordnet ist, das so gesteuert wird, daß es während der Inspiration geschlossen ist, wobei die Vorrichtung außerdem Steuermittel (12), die mit mindestens einem Meßfühler (18, 19) verbunden sind, der die Atemtätigkeit des Patienten erfaßt, und Verteilermittel ($V_I$) umfaßt, die in der Inspirationsphase die Verbindung zwischen der Inspirationsförderstromquelle (11) und dem Inspirationszweig (3) des Patientenkreislaufs herstellen und in der Exspirationsphase diese Verbindung wenigstens teilweise unterbrechen, dadurch gekennzeichnet, daß die Verteilermittel ($V_I$) von den Steuermitteln (12) gesteuert werden, welche die Unterbrechung der Verbindung befehlen, wenn der Meßfühler feststellt, daß der Patient eine Exspirationsphase vorbereitet, und daß die Steuermittel (12) außerdem wenigstens indirekt das Exspirationsventil ($V_E$) steuern, damit dieses einen vordefinierten Exspirationsdruck bestimmt, der im wesentlichen unabhängig von dem Betriebszustand der Inspirationsförderstromquelle (11) ist.

2. Vorrichtung nach Anspruch 1, dadurch gekennzeichnet, daß die Inspirationsförderstromquelle eine Motorturbine (11) ist.

3. Vorrichtung nach Anspruch 1 oder 2, dadurch gekennzeichnet, daß die Inspirationsförderstromquelle (11) während der Exspirationsphasen in der den Inspirationsförderstrom liefernden Richtung in Betrieb gehalten wird.

4. Vorrichtung nach einem der Ansprüche 1 bis 3, dadurch gekennzeichnet, daß die Inspirationsförderstromquelle (11) so betrieben wird, daß sie einen Ausgangsdruck liefert, der während der Inspirationsphasen und während der Exspirationsphasen im wesentlichen gleich ist.

5. Vorrichtung nach einem der Ansprüche 1 bis 4, dadurch gekennzeichnet, daß die Inspirationsförderstromquelle (11) von einer Art ist, die einen Förderdruck aufweist, der über einen Förderstromwertebereich, der die Förderströme während der Inspirationsphasen und die verschiedenen Förderströme während der Exspirationsphasen einschließt, in derselben Größenordnung bleibt.

6. Vorrichtung nach Anspruch 5, dadurch gekennzeichnet, daß die verschiedenen Förderströme einen Förderstrom von im wesentlichen Null einschließen.

7. Vorrichtung nach einem der Ansprüche 1 bis 4, dadurch gekennzeichnet, daß die Inspirationsförderstromquelle (11) eine Quelle ist, deren Förderstrom gegen Null geht, wenn der Druck an ihrem Ausgang einen maximalen Wert annimmt, der in der Größenordnung eines Beatmungsdruckes liegt.

8. Vorrichtung nach einem der Ansprüche 1 bis 7, dadurch gekennzeichnet, daß die Verteilermittel ein Inspirationsventil ($V_I$) mit pneumatischer Steuerung umfassen, dessen Steuereingang (41) selektiv während der Exspiration mit dem Ausgang der Inspirationsförderstromquelle (11) und während der Inspiration mit einem geringeren Druck (43) verbunden wird.

9. Vorrichtung nach Anspruch 8, dadurch gekennzeichnet, daß der geringere Druck der Gasbahn zwischen dem Inspirationsventil ($V_I$) und dem Patientenkreislauf (1) entnommen wird.

10. Vorrichtung nach einem der Ansprüche 1 bis 9, dadurch gekennzeichnet, daß die verteilermittel dafür ausgelegt sind, während der Exspiration zwischen der Inspirationsförderstromquelle (11) und dem Patientenkreislauf (1) einen Förderstrom zum Verlustausgleich (44) bestehen zu lassen.

11. Vorrichtung nach einem der Ansprüche 1 bis 9, dadurch gekennzeichnet, daß die Verteilermittel ($V_I$) von einer Zweigleitung (53, 54) umgangen werden, die mit einer Verengung (52) und, stromabwärts von dieser Verengung, mit einem zusätzlichen Ventil (VR) versehen ist, das so gesteuert wird, daß es während der Inspiration geschlossen ist und während der Exspiration in der Zweigleitung stromabwärts von der Verengung (52) einen Druck nahe dem vordefinierten Exspirationsdruck (PEP) aufrechterhält.

12. Vorrichtung nach einem der Ansprüche 1 bis 11, dadurch gekennzeichnet, daß das Exspirationsventil ($V_E$) pneumatisch gesteuert wird und daß zur Betätigung des Exspirationsventils ($V_E$) die Steuermittel die Aktivierung einer Verbindung (7, VC1) zwischen einem Steuereingang (6) des Exspirationsventils ($V_E$) und einer Niederdruckquelle (9, 15) befehlen.

13. Vorrichtung nach Anspruch 12, dadurch gekennzeichnet, daß die Verbindung eine Ventileinrichtung (VC1) enthält, die in einem Steuerkreis (7) des Exspirationsventils ($V_E$) angeordnet ist und von den Steuermitteln (12) so gesteuert wird, daß sie während der Exspiration des Patienten den Steuereingang (6) des Exspirationsventils mit der Niederdruckquelle (14) verbindet.

14. Vorrichtung nach Anspruch 13, dadurch gekennzeichnet, daß die Ventileinrichtung ein Dreiwegventil (VC1) umfasst , das selektiv den Steuereingang (6) des Exspirationsventils ($V_E$) mit der Druckförderstromquelle (11) und mit der Niederdruckquelle (14) verbindet.

15. Vorrichtung nach einem der Ansprüche 12 bis 14, dadurch gekennzeichnet, daß die Niederdruckquelle eine Verbindung (14) zwischen einer Auslaßdüse (17) und einem Ausgang einer zweiten Förderstromquelle (16) umfaßt.

16. Vorrichtung nach Anspruch 15, dadurch gekennzeichnet, daß die zweite Förderstromquelle einen Kompressor (16) umfaßt.

17. Vorrichtung nach Anspruch 15, dadurch gekennzeichnet, daß die zweite Förderstromquelle eine Vorrichtung (15) zur Verbindung mit der Inspirationsförderstromquelle (11) umfaßt.

18. Vorrichtung nach einem der Ansprüche 1 bis 17, dadurch gekennzeichnet, daß der vordefinierte Exspirationsdruck einstellbar ist und einen Wert gleich dem Atmosphärendruck annehmen kann.

19. Vorrichtung nach einem dei Ansprüche 1 bis 18, dadurch gekennzeichnet, daß die Steuermittel (12) die Schließung des Exspirationsventils ($V_E$) befehlen, wenn sich der Druck in dem Patientenkreislauf (1) am Ende der Exspirationsphase des Patienten verringert.

20. Vorrichtung nach Anspruch 19, dadurch gekennzeichnet, daß die Steuermittel (12) empfindlich für die Ableitung des Druckes in dem Patientenkreislauf (1) nach der Zeit sind.

21. Vorrichtung nach einem der Ansprüche 1 bis 20, dadurch gekennzeichnet, daß sie Mittel (18, 19) zur Messung des Druckes (P) und des Förderstromes (D) in dem Inspirationszweig (3) des Patientenkreislaufs (1) und Mittel zur Berechnung des Volumens des eingeatmeten Gases oder einer entsprechenden Größe umfaßt.

22. Vorrichtung nach einem der Ansprüche 1 bis 21, gekennzeichnet durch Mittel (12) zur Aktivierung eines Loslösungsalarmes (49), wenn der Förderstrom in dem Inspirationszweig (3) während einer vorbestimmten relativ großen Zeitdauer hoch bleibt.

FIG_1

ELECTRONIQUE DE COMMANDE — 12

11

SOURCE DE DEBIT INSPIRATOIRE

$V_I$   18   D   P — 19   3   1

10   4   6   $V_E$   8   patient   2

DEUXIEME SOURCE DE DEBIT

16   9   15   14   13   17   VC1   7

FIG_2

INITIALISATION

$P_1 = 0$
$T_1 = 0$

REINITIALISER COMPTAGE TEMPS $T_{INS}$

$D_{MAX} = 0$
$Q = 0$

OUVERTE   $V_I$   FERMEE   21

$T_2 = T_{INS}$
$P_2 = P$   29

NON   $D > D_{MAX}$ ?   23   NON   $\dfrac{P_2 - P_1}{T_2 - T_1} < A?$   OUI   31

24   $D_{MAX} = D$   $Q = 0$   OUI   $Q = 0$ ?   NON   33

26   22   $T_A = T_2$   $Q = 1$   32   NON   $T_{INS} - T_A > D_A$ ?

$D < K.D_{MAX}$ ?   NON   34   36

OUI   OUI   $T_{INS} > T_{MAX}$ ?   OUI   $T_{INS} > T_F$   OUI   OUI

27   28   NON   NON

FERMER $V_I$
ACTIONNER $VC_1 \rightarrow BP$   $P_1 = P_2$
$T_1 = T_2$   OUVRIR $V_I$
ACTIONNER $VC_1 \rightarrow HP$

48   $F = 60/T_{INS}$

FIG_3

FIG_4

FIG.5